# EUROPEAN PATENT SPECIFICATION

(11) **EP 0 666 927 B1**
(45) Date of publication and mention of the grant of the patent: **07.05.1997**
(21) Application number: 92919473.6
(22) Date of filing: 14.09.1992
(51) Int. Cl.: C12Q 1/68

(54) **Method of detecting a nucleic acid sequence**
Verfahren zur Bestimmung von einer Nukleinsäuresequenz
Méthode de détection d'une séquence d'acide nucléique

(30) Priority: 13.09.1991 GB 9119650; 27.09.1991 GB 9120656
(43) Date of publication of application: 16.08.1995
(73) Proprietor: CYTOCELL LIMITED, Adderbury, Banbury, Oxon OX17 3SN (GB)
(72) Inventor: CARDY, Donald, Leonard, Nicholas, Aston-le-Walls Northamptonshire NN11 6UF (GB); DELNATTE, Sabine, Yolande, Joseph, Lewknor Oxfordshire OX9 5TS (GB)
(74) Representative: Matthews, Heather Clare
(86) International application number: GB9201680
(87) International publication number: WO9306240

(56) References cited:
- EP-A- 0 361 983
- EP-A- 0 419 081
- EP-A- 0 427 073
- EP-A- 0 427 074

## Description

### Field of the Invention

This invention relates to a method of detecting a nucleic acid sequence of interest and kits for performing the method of the invention.

### Background of the Invention

Processes for direct amplification of specific nucleic acid sequences are known and have been described in the prior art. Kleppe et al., in J. Mol. Biol. 56 (1971) p341-361, disclose the use of nucleic acid primers which hybridise adjacent to a specific nucleic acid sequence of interest. The primers are annealed to opposite strands of a denatured DNA duplex and extended using DNA polymerase and nucleoside triphosphates to give two duplex molecules of the original nucleic acid sequence. Successive cycles of denaturation, annealing and extension are undertaken in order to further amplify copies of the original nucleic acid sequence.

The above method is now referred to as the polymerase chain reaction (PCR) and is further described in US4683195 and US4683202 where extension of the annealed nucleic acid primers is effected either with Thermus aquaticus (Tag) DNA polymerase or the Klenow fragment of DNA polymerase I. Disadvantages of this method include the need for adjusting reaction temperatures alternatively between intermediate (e.g. 55-60°C) and very high (e.g. 90-95°C) temperatures (involving repeated "thermal cycling" to high temperatures), the extended timescale needed for multiple cycles of large temperature alterations to achieve amplification of a nucleic acid sequence and the occurrence of sequence errors in the amplified copies of the nucleic acid sequence due to errors arising during multiple copying of long tracts of sequence. Additionally, one or more further processes are invariably required (e.g. gel electrophoresis) for detection of the amplified nucleic acid sequence.

Alternative nucleic acid amplification processes are disclosed in WO88/10315 (Siska Diagnostics) and EP329822 (Cangene) and EP373960 (Siska Diagnostics) whereby a cycling reaction comprising alternative DNA and RNA of oligonucleotides adjacent to a specific DNA sequence whereby these oligonucleotides comprise a transcriptional promoter and initiation site. The RNA copies of the specific sequence so produced, or alternatively an input sample comprising a specific RNA sequence, are then copied as DNA strands using a nucleic acid primer and the RNA from the resulting RNA:DNA hybrid is either removed by denaturation (WO 88/10315) or destroyed using RNase H (EP329822 and EP 373960). The annealing of oligonucleotides forming a transcriptional promoter is then repeated in order to repeat RNA production. Amplification is achieved principally through the use of efficient RNA polymerases to produce an excess of RNA copies over DNA templates at each cycle. The version of this method including RNase H has the advantage over PCR that amplification can potentially be achieved at a constant ambient temperature. In addition, whilst PCR results in a doubling of DNA copies at each cycle, DNA:RNA cycling can potentially achieve a much greater amplification per cycle, for example 10-100 RNA copies per cycle using T7 RNA polymerase. A disadvantage of the DNA:RNA cycling method described in EP329822 is that it requires test nucleic acid with known, discrete ends for the annealing of oligonucleotides to create the transcriptional promoter. This poses difficulties in detection of, for example, specific genes in long DNA molecules. Further disadvantages of this method are that at least 3 enzymes are required to undertake the DNA:RNA cycling with potentially deleterious consequences for stability, cost and reproducibility; and that one or more further processes are invariably required (e.g. gel electrophoresis) for detection of the amplified nucleic acid sequence.

The processes described above all refer to methods whereby a specific nucleic acid region is directly copied and these nucleic acid copies are further copied to achieve amplification. The variability between different nucleic acid sequences is such that the rates of amplification between different sequences by the same process are likely to differ thus presenting problems for example in quantitating the original amount of specific nucleic acid. Other processes do not require the copying of specific test nucleic acid. WO 87/06270 describes the use of RNA probes including sequences for amplification by RNA-dependant RNA polymerases. In particular, the specification describes autocatalytic replication by the bacteriophage Q-beta replicase. The use of sequence-specific RNA probes with an adjacent promoter for autocatalytic RNA amplification has the advantage of potentially "uniform" amplification of the hybridised probe itself rather than different nucleic acid sequences but disadvantages include the need to remove unhybridised probe molecules before amplification and the fact that a hybridisation signal is dependent on only a single hybridisation (with likely increases in "background noise"). EP 320308 describes the use of pairs of complementary adjacently hybridising nucleic acid probes which are then ligated together at the hybridisation site to produce a longer probe. In successive cycles of denaturation, reannealing and ligation, both the specific target nucleotide sequence and previously ligated probes can act as templates for formation of additional ligated probes. Thus the target sequence is not itself amplified but gives rise to formation of ligated probes which themselves act as substrates for further ligations. This process has the disadvantages of likely ligation of single-stranded ends ("sharp-end ligation") and absolutely requires one or more extra processes (e.g. gel electrophoresis) in order to detect ligated probe molecules.

### Summary of the Invention

In one aspect the invention provides a method of testing for the presence of a nucleic acid sequence of interest in a sample, comprising: contacting the sample with a first probe and a second probe, said probes being capable of hybridising to the sequence of interest such that the probes are adjacent or substantially adjacent to one another and with portions of the probes non-complementary to the sequence of interest being annealed at last in part; treating the sample so as to cause chain extension of at least one of the probes; and detecting the extended probe.

Another embodiment is envisaged according to the method defined above, further comprising the use of a third probe and a fourth probe, portions of said third and fourth probes being able to hybridise respectively to at least part of the sequences of the first and second probes complementary to the sequence of interest, so as to enable other portions of the third and fourth probes to anneal to each other; and treating the sample so as to cause chain extension of the third or fourth probe using part of the third or fourth probe as a template.

Generally, it is envisaged that the first probe comprises a sequence substantially complementary to the sequence of interest and further comprises a sequence substantially non-complementary to the sequence of interest. Typically these will contain 15-25 and 2-10 nucleotides respectively. It is further envisaged that generally the second probe comprises a sequence substantially complementary to the sequence of interest and further comprises a sequence which is substantially non-complementary to the sequence of interest but which is substantially complementary to at least part of that sequence of the first probe which is non-complementary to the sequence of interest. Typically these regions of the second probe will contain 15-25 and 30-70 nucleotides respectively. Typically therefore, the second probe is longer than the first probe and may act as a template for the extension of the first probe.

As a particular variant of the above, one of the probes may contain self-complementary nucleotides and may therefore be able to self-prime for extension, with the same probe acting as a template. This may then be ligated to the other probe, which is held in proximity to the extended probe by virtue of their common hybridisation to the sequence of interest. Annealing of those portions of the probes which are non-complementary to the sequence of interest is achieved via a comparatively short region of complementarity between the probes. As will be apparent to those skilled in the art, hybridisation conditions can be readily selected such that the two probes will only anneal if stabilised in proximity to each other by prior hybridisation to the sequence of interest ("target" sequence). Thus the extended probe will only be formed in the presence of the sequence of interest.

The presence of the extended probe can be detected directly by, for example, conventional methods (e.g. by binding sequence specific nucleic acids, proteins or other substances or by gel electrophoresis size analysis for the extended probe ). Generally however, further processing of this newly-synthesised DNA is desirable to achieve amplification of one extended probe sequence.

Various methods of amplifying the extended probe are envisaged. These of course include prior art techniques such as polymerase chain reaction (PCR). However, other novel techniques are also envisaged.

In another aspect the invention provides a method of amplifying an extended probe produced as defined above, comprising:
hybridising to an extended first probe a further probe substantially complementary to at least part of the newly-synthesised sequence of the extended first probe;
extending the further probe by use of an appropriate polymerase using the extended first probe as a template;
and separating the extended first and further probes, such that the extended further probe can act as a template for the extension of other first probe molecules and the extended first probe can act as a template for the extension of other further probe molecules.

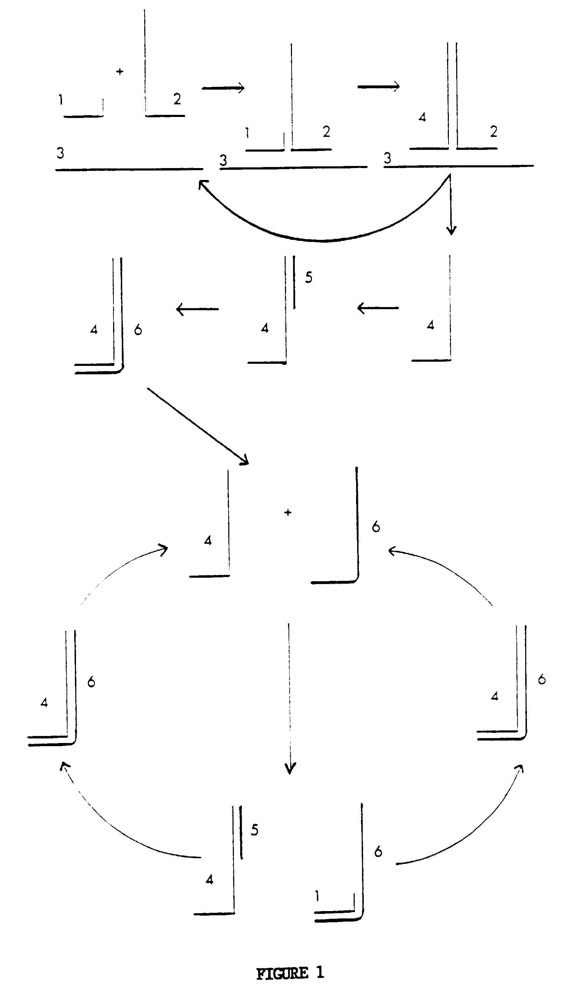

This aspect of the invention is described further below, with reference to the lower portion of Figure 1.

Generally the further probe is an oligodeoxyribonucleotide which may be extended by a DNA polymerase, typically Taq polymerase.

As an alternative to the use of a further probe, the extended first probe may contain self-complementary sequences at the 3' end. Thus upon separation from the template strand the extended first probe may self-anneal and thereby self-prime for its own extension.

In this way, by repeated cycles of annealing, extension and denaturation, amplification of the original extended probe can be achieved. Preferably denaturation is achieved by moderate heating.

In one preferred method of amplification, parts of the newly extended probe and the complementary probe used as template comprise a region of double stranded nucleic acid recognised by a polymerase, most preferably an RNA polymerase. Efficient RNA polymerases such as T7 or SP6 polymerase may then produce large quantities of RNA from one of the strands of the double stranded nucleic acid (the "sense" strand). Conveniently, the newly-extended probe is the sense strand.

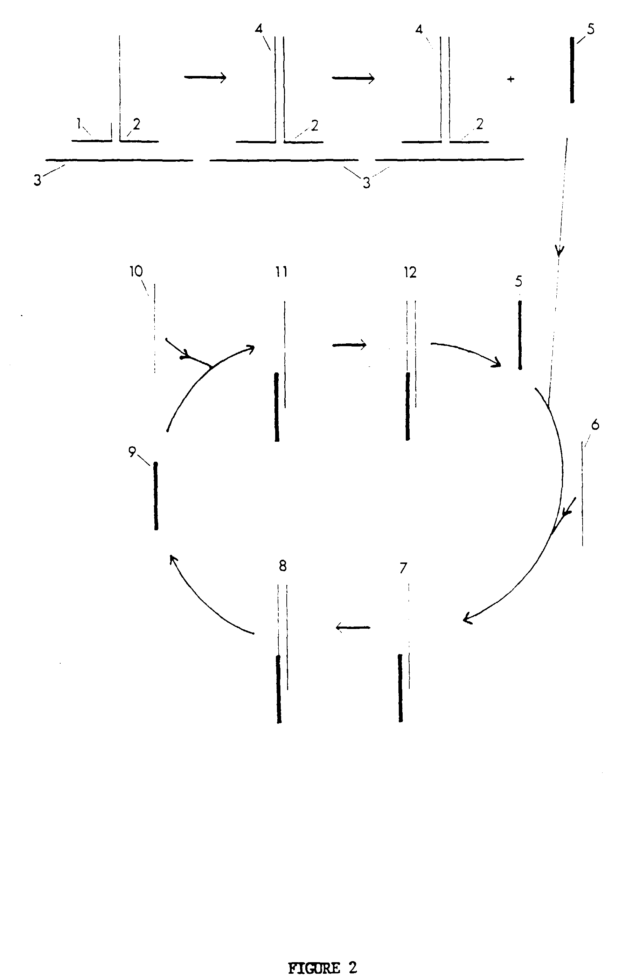

As with the extended probe sequence, the RNA copy may be detected directly, for example, by conventional techniques. Alternatively, it may be convenient to further amplify the RNA copy, for example, to improve sensitivity. One method of amplifying the RNA is further described below, with reference to Figure 2. In this embodiment, the RNA molecule is extended after annealing a single stranded DNA molecule which is at least partly complementary to the RNA copy, and thus can act as a template. The new double stranded nucleic acid molecule which results comprises a recognition site for an RNA polymerase. Preferably, this recognition site should be recognised by an RNA polymerase different to the one responsible for making the original RNA copy. This enables RNA copies to be made. These RNA copies can in turn be extended after annealing to a single stranded DNA molecule which is at least partly complementary to the RNA molecule. The new double stranded nucleic acid molecule which results comprises a recognition site for an RNA polymerase which, in the preferred embodiment enables RNA copies to be made which have the same sequence as the original RNA molecule which started the process. This is clearly an efficient way of amplifying the RNA copy.

A further specific embodiment is envisaged wherein the original RNA molecule produced comprises a region which can be replicated and amplified by Q-beta replicase. Thus multiple copies of the molecule can be formed.

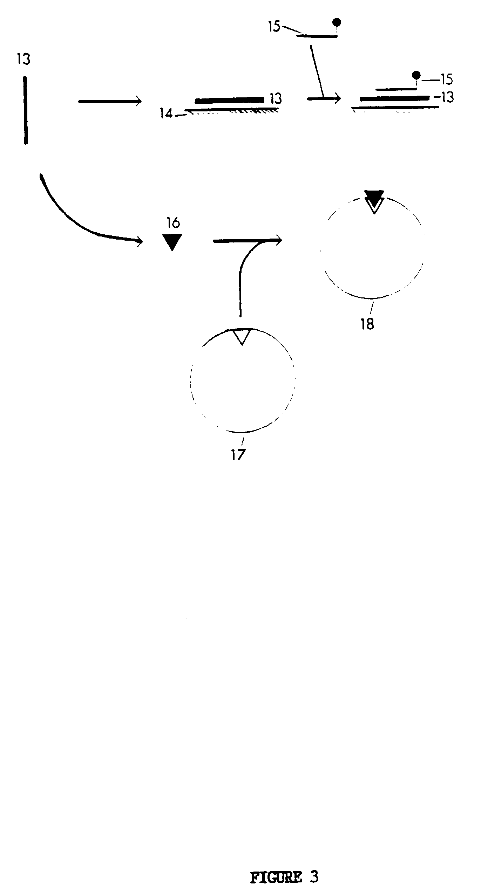

Whether or not the RNA copies are amplified, they may be detected in various ways, for example, by hybridisation to sequence specific labelled probes, or other conventional methods. Alternatively, as described in greater detail below, with reference to Figure 3, the RNA copy may contain the necessary translation start and stop signals to allow reasonably efficient translation into an amino acid sequence, the presence of which can be readily detected due to some particular characteristic of the amino acid sequence.

In another aspect therefore the invention provides a method of detecting the presence of an RNA molecule (comprising translation start and stop codons), comprising: translating of the RNA molecule into an amino acid sequence having a particular characteristic; and detecting the amino acid sequence. The RNA sequence for translation has a number of preferred features: its length is conveniently 100-200 nucleotides. The translation initiation codon should preferably be situated between 10 and 30 nucleotides from the 5' end, which end conveniently comprises a triphosphate-linked 7-methylguanosine residue.

Conveniently the translation is effected using components provided by rabbit reticulocyte or wheat-germ lysates, or other preparations with high translation efficiency.

Preferably the amino acid sequence acts as an enzyme activator, cofactor or repressor. Conveniently, the amino acid sequence comprises an N terminal fragment of B-galactosidase. Thus by the addition of the rest of B-galactosidase an enzyme activity will be formed. This can be detected in a manner well known to those skilled in the art (e.g. by the use of enzyme substrates giving rise to coloured products).

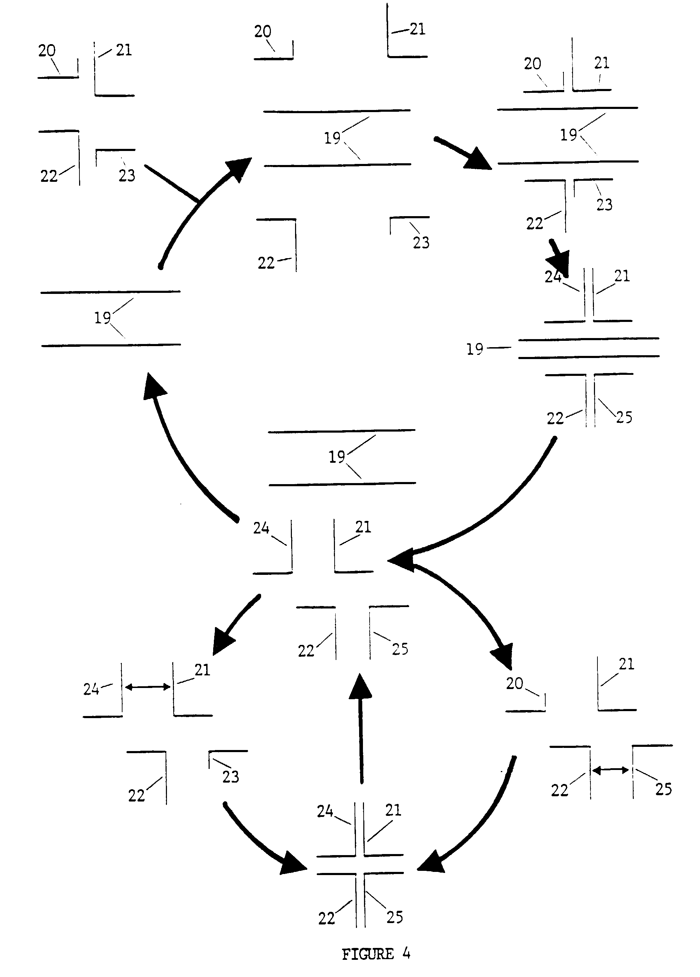

Other methods of amplifying the extended probe of the invention are envisaged. These include the use of 2 pairs of probes, as described in further detail below, with reference to Figure 4. In this embodiment a first and second probe hybridise to a nucleotide target sequence so as to be adjacent or substantially adjacent such that one probe of the pair may be extended using the other probe as a template. Following extension, the first and second probes may be separated from the target sequence (e.g. by heat denaturation) yet may themselves form a stable hybrid by reason of the extended complementary sequence. Third and fourth probe molecules may then become hybridised to the stable hybrid of first/second probes so as to be adjacent or substantially adjacent via sequences complementary to the target specific regions of the first and second probes respectively. Thus one of the pair of third/fourth probes may become extended using the other probe as a template as before. This extension allows the third and fourth probes to form a stable hybrid which may therefore act as a target sequence for further molecules of the first and second probes, leading to their hybridisation and extension. Thus in successive cycles of denaturation and renaturation, amplification of the original extended probe sequence may be achieved.

As a variant of this embodiment, the target nucleotide sequence may be double stranded and the first/second and third/fourth probes bind to opposite strands of the target. In this way one probe from both pairs may be extended during the same cycle.

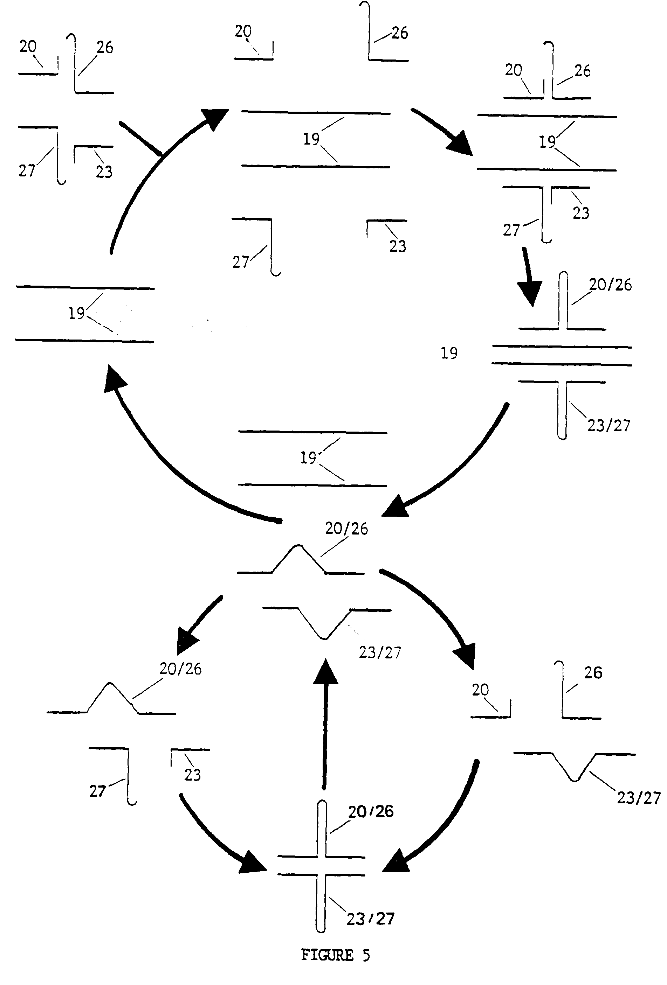

A further variant is described below in relation to Figure 5. To this further embodiment first and second probes may hybridise to the sequence of interest so as to be adjacent or substantially adjacent, as before. However, one of the pairs of probes contains a "hairpin loop". Thus, following extension of one of the probes they may be treated with a DNA ligase to produce a single looped molecule. As before, third and forth probes may also be present which hybridise either to the other strand of the nucleotide target sequence or to the single looped molecule hybrid of the first and second probes. The hybridisation brings the third and fourth probes adjacent or substantially adjacent, thereby allowing priming and extension. In the same way as described previously, inclusion of a hairpin loop at one end of the probe molecules allows for treatment with DNA ligase to form a single looped molecule hybrid of the third and fourth probes.

These hybrid molecules may then hybridise to unmodified pairs of probes leading to extension and ligation of the unmodified probes. Thus by successive cycles of denaturation and renaturation, amplification of the originally extended probe sequence can be achieved.

In yet another aspect, the invention provides for use of a kit for performing a method of the invention, comprising a polymerase and instructions for use.

Preferably the probes which anneal ("first" and "second" probes) to the target sequence are oligodeoxyribonucleotides, typically each including between 15 and 25 nucleotides complementary to the target nucleotide sequence (i.e. the sequence of interest) although they may contain fewer or more complementary nucleotides.

Preferably the portion of the second probe which is non-complementary to the target sequence is 30-70 nucleotides long, and typically is at the 5' end of the probe.

Generally, the target non-complementary (but second probe-complementary) sequence of the first probe is 2-10 nucleotides long, and is typically at the 3' end of the probe. The non-complementary sequences of both probes may be longer, and that of the second probe may be shorter.

It is an essential feature of the invention that the first and second probes, when hybridised to the target sequence, are adjacent or substantially adjacent to each other. Use of the term "adjacent" is intended to mean flat there are no nucleotides of the target sequence left without base-pairing between those portions of the target sequence ("loci") which are base-paired to the complementary sequence of the probes. This proximity between the probes enables the target-non-ccmplementary sequences of the probes to anneal. As will readily be apparent to those skilled in the art, by designing the probes so as to allow for annealing to each other at greater separations from the target sequence, gaps may be introduced between the loci in the target nucleotide sequence to which the probes hybridise. In this situation the probes are said to be "substantially adjacent", because there may be some nucleotides of the target sequence left without base-pairing between those portions of the target sequence which are base-paired to the probes. Clearly, the number of intervening un-paired nucleotides of the target sequence can vary according to the design of the probes. Thus whilst it is preferred that the first and second probes hybridise so as to be adjacent, the probes may be separated by up to 5 nucleotides of target sequence.

In a preferred embodiment primer extension of the second probe (using the target sequence as a template) can be prevented by 'blocking' the 3' end of the probe. This may be conveniently done by the inclusion at the 3' end of, for example, a biotinylated or a thiolated nucleotide, or a dideoxynucleotide.

Conveniently, the first and second probes are DNA and the first probe is extended by a DNA polymerase activity. Suitable enzymes include AMV reverse transcriptase, the Klenow fragment of E. coli DNA polymerase I or Taq polymerase.

Further methods of amplification are envisaged by the present inventors. Thus in one embodiment, both probes are DNA and extension of the first probe results in the creation of newly-synthesized double-stranded DNA (ds DNA), said ds DNA comprising a promoter and transcription initiation site for transcription by an RNA polymerase. Addition of a suitable RNA polymerase (such as T7 or SP6 polymerase) thereby produces first RNA copies of the 'sense' (+) strand.

Preferably the extended first probe is the sense strand. Conveniently said first RNA copies comprise 20-40 ribonucleotides although they may contain fewer or more ribonucleotides, as will be apparent to those skilled in the art.

As will be obvious to those skilled in the art, the extended probe may be subjected to amplification processes, such as those previously described, to produce DNA copies, which DNA copies may then be in turn amplified as RNA molecules, using those methods described herein or known from the prior art.

It will be appreciated that the primer extension product of the method of the invention, or the RNA copies produced therefrom may be further amplified by processes already known in the prior art. For example, the primer extension product may be subject to the PCR method described in US4683195 and US4683202, or may be subject to alternative DNA and RNA cycling as disclosed by EP329822, or may be subject to ligation of adjacently hybridised probes as claimed in EP320308.

The process of the current invention may be effected on a solid phase whereby the target nucleic acid is attached to the solid phase, for example a membrane or a bead, or is in the liquid phase, (e.g. in solution). Solid phases may also be used with alternative aspects of the invention, for example where a single-stranded DNA molecule primed by an amplified RNA is attached to a solid phase. The process of the current invention may be used in conjunction with a range of methods for detecting either the primer extension product or RNA copies produced therefrom. For example, nucleic acid products may be detected by the hybridisation of a labelled nucleic acid probe or by size analysis of nucleic acid products by gel electrophoresis.

The process of the current invention is applicable to the detection of any DNA or RNA target sequence and to the detection of lesions in genetic material such as deletions and translocations. Through the use of small nucleotide probes, the process of the current invention is particlularly suitable for the detection of point mutations or single base polymorphisms in genetic material whereby stringent hybridisation conditions can readily be achieved such that normal hybrid formation is precluded by a single base mismatch between target sequence and probe. The process of the current invention is applicable to all areas of diagnostic medicine, such as detection of microorganisms and detection of genes associated with genetic disease, and other diagnostic sciences such as veterinary, agricultural, forensic, food analysis and molecular biology research.

Thus, depending on the use to which the invention is applied, the "sequence of interest" may be very long (for example, an entire gene) or may be rather short (for example, in the detection of point mutations). Thus the probes defined previously may hybridise to the target sequence such that they are joined to just a small length of the sequence of interest or they may be joined such that some of the target sequence is beyond the sequence which is strictly of interest.

The various aspects of the invention will be better understood by reference to the following illustrative examples and drawings, of which:-
Figures 1-5 are schematic representations of methods according to the invention and
Figures 6-8 show autoradiographs of results obtained from experiments performed using methods according to the present invention.

### Description of Specific Embodiments

In one embodiment of the invention, shown schematically in Figure 1, a first probe 1 and a second probe 2 hybridise (substantially adjacent to each other) via target-complementary sequences to a sequence of interest 3.

This arrangement allows for probe 1 to be primer extended, due to a short region of complementarity between probes 1 and 2, which is non-complementary to the target nucleic acid and therefore does not hybridise to the sequence of interest 3. This extension utilises 2 as a template and can be effected by any suitable RNA or DNA polymerase, depending on the nature of the probes. Probe 2 cannot be extended (using the target as a template) because it is blocked at the 3' end (for example by a terminal thionucleotide, deoxyribonucleotide or a biotinylated nucleotide). Thus primer extension of probe 1 results substantially in the production of extended probe 4.

Following denaturation, the target nucleotide sequence 3 is released for further hybridisation by probes 1 and 2 whilst the newly-added unmodified third oligonucleotide probe 5 hybridises to the primer extended molecule 4 and can then itself be extended by the DNA polymerase to produce an extended molecule 6. Following denaturation, molecules 4 and 6 are then released for further hybridisation by probes 5 and 1 respectively which can then be extended to produce additional molecules of 6 and 4 respectively. These can be subject to further cycles of denaturation, hybridisation and primer extension. Thus, at each cycle, one new molecule 4 is produced as a result of hybridisation of 1 and 2 to target nucleic acid 3, one new molecule 6 is produced as a result of hybridisation and extension of 5 on a template of molecule 4 from a previous cycle, and one new molecule each of 4 and 6 are produced as a result of extension of 5 and 1 respectively on molecules of 4 and 6 respectively from previous cycles.

Another specific embodiment is illustrated schematically in Figure 2. Probes 1 and 2 hybridise to a target sequence 3 as described previously, allowing for extension of 1 by a DNA polymerase to give an extended probe 4, such that a new double-stranded DNA region is formed from 4 and 2 which comprises a promoter and transcriptional initiation site for the action of an RNA polymerase which can then produce RNA copies 5. The RNA copies 5 can then anneal through a short region of complementarity to a single-stranded DNA molecule 6 to give a hybrid RNA/DNA molecule 7 which can then be primer extended from the RNA strand to produce a new double-stranded DNA region in molecule 8 which comprises a promoter and transcriptional initiation site for the action of a RNA polymerase which then produces RNA copies 9. The said RNA copies 9 can then anneal through a short region of complementarity to a second single-stranded DNA molecule 10 to give hybrid RNA/DNA molecule 11 which can then be primer extended from the RNA strand to produce a new double-stranded DNA region in molecule 12 which comprises a promoter and transcriptional initiation site for the action of a RNA polymerase which then produces further RNA copies 5 which re-enter the cycle of RNA:DNA annealing, primer extension and production of further RNA copies 9 and 5.

Figure 3 illustrates schematically two alternatives for measurement of RNA copies produced as a result of the methods of the invention. In one alternative, RNA copies 13 are immobilised onto a solid phase 14 and detected by hybridisation to labelled probe 15 to produce a hybrid of 13 and 15 and thus the association of the label with the solid phase. In another alternative, RNA copies 13 comprise translational initiation and termination codons together with a 5' triphosphate-linked 7-methylguanosine residue which promotes efficient translation of RNA copies 13 into a peptide fragment of beta-galactosidase 16 which complements an inactive enzyme fragment 17 to yield active beta-galactosidase 18. Clearly, other enzymes might be substituted in the scheme for beta-galactosidase.

A further embodiment envisaged within the scope of the present invention is shown schematically in Figure 4. The method is essentially similar to those described previously except that the target sequence 19 is double-stranded and two pairs of probes, 20, 21 and 22, 23, are employed.

Thus, oligonucleotide probes 20 and 23 and the 3' blocked oligonucleotide probes 21 and 22 hybridise substantially adjacently to their respective partners on a double-stranded target nucleotide sequence 19 such that said probes 20 and 23, through a short region of complementarity with probes 21 and 22 respectively, can be extended using a DNA polymerase to produce primer extended molecules 24 and 25. Following denaturation, target nucleotide sequence 19 is released for further hybridisation by probes 20 to 23 whilst primer extended molecules 24 and 25 can rehybridise to molecules 21 and 22 respectively due to the newly extended regions of complementarity. These rehybridised molecules 21/24 and 22/25 now comprise target nucleotide sequences for hybridisation of molecules 22/23 and 20/21 respectively. These hybridised molecules 20 and 23 can now be extended by the DNA polymerase to produce additional molecules 24 and 25 are released for further hybridisation by molecules 21 and 22 respectively thus continuing a cycle of replication of molecules 24 and 25. Thus, at each cycle, one new molecule 24 and 25 is produced as a result of hybridisation of 20 and 21 to target nucleic acid 19 and a further new molecule of 24 and 25 is produced as a result of hybridisation and extension of 20 and 23 on a template of molecules 22/25 and 21/24 respectively as formed in a previous cycle.

A further embodiment is illustrated schematically in Figure 5, wherein oligonucleotide probes 20 and 23 and the 3' blocked oligonucleotide probes 26 and 27, (which both comprise short 5' hairpin loops), hybridise adjacently to a double-stranded target nucleotide sequence 19 such that said probes 20 and 23, through a short region of complementarity with probes 26 and 27 respectively, can be extended using a DNA polymerase and ligated using a DNA ligase to produce looped molecules 20/26 and 23/27. Following denaturation, these additional molecules 20/26 and 23/27 are released for further hybridisation, thus continuing a cycle of replication of looped molecules 20/26 and 23/27. Thus, at each cycle, one new looped molecule of 20/26 and 23/27 is produced as a result of hybridisation to target nucleic acid 19 and a further new looped molecules are produced as a result of hybridisation, extension and ligation to form 20/26 and 23/27 on templates of looped molecules 23/27 and 20/26 respectively as formed in a previous cycle.

### Examples

The general methods for production and use of nucleic acid probes were as detailed in "Molecular Cloning, A Laboratory Manual", eds. Sambrook, Fritsch and Maniatis, Cold Spring Harbor Press (1989) which will be hereafter referred to as "Molecular Cloning". The oligonucleotides used in the examples are detailed in table 1. Unless otherwise specified, all oligonucleotides were synthesised on an Applied Biosystems 380A synthesiser using long-chain alkylamino CPG supports and nucleoside phosphoramidites supplied by Cruachem (Glasgow, UK) and used in accordance with manufacturer's instructions. All oligonucleotides were HPLC purified as described in "Molecular Cloning" and were finaly lyophilised and dissolved in water at 10pmoles/µl.

### Example 1 - Hybridisation and Extension of Probe for CFTR Gene

This example demonstrates the production of a new nucleic acid strand as a result of the interaction of adjacently hybridised probes for the CFTR (cystic fibrosis transregulator) gene and the effect of hybridisation to the delta-508 deletion of this gene associated with the disease of cystic fibrosis.

### I. Preparation of Oligonucleotides

Oligos 1 and 2 were synthesised with a 3'-thiol group by the method of Zuckermann et al. (Nucleic Acids Research, 15 (1987) p5305-5321) or, alternatively, oligo 1 was purchased with a 3'-biotin terminus from a commercial source (Severn Biotech, Kidderminster, UK) and purified as above.

### II. Preparation of Target DNA

DNA samples were obtained from a normal individual homozygous for the non-mutated CFTR gene and from an individual afflicted with cystic fibrosis by virtue of a homozygous deletion of codon 508 in the CFTR gene. DNA was derived from buccal cells obtained from these individuals by gentle scraping of the buccal lining with a sterile toothpick. Buccal cells were then suspended in 10µl sterile water, lysed with 20µl 0.1M potassium hydroxide and 0.1% Triton®-X-100 at 65°C for 20 minutes, and neutralised with 20µl of 0.1M HCl and 0.1% Triton®-X-100.

Regions of the CFTR gene were then amplified by the polymerase chain reaction (PCR) procedure as described in EP200362 (Cetus Corporation). 5µl of the human cell lysate was denatured at 94°C for 3 minutes and mixed into 5µl of 20mM Tris.HCl pH8.3, 100mM potassium chloride, 3mM magnesium chloride, 20ng/ml bovine serum albumin (fragction V, Sigma, Poole, UK), 400µM deoxyribonucleotide (dNTP) mixture (mixture of deoxyadenosine triphosphate, deoxythmidine triphosphate, deoxyguanosine triphosphate and deoxycytidine triphosphate (dCTP), all obtained from Pharmacia, Milton Keynes, UK), 0.1% Triton®-X-100, 0.05 units Thermus aquaticus DNA polymerase (United States Biochemicals, Cleveland, Ohio, USA) and 0.5µM oligonucleotide amplification primers 5'-CAGTGGAAGAATGGCATTCTGTT-3' and 5'-GGCATGCTTTGATGACGCTTCTG-3'. Amplification was undertaken by 40 cycles in a thermal cycler (Techne, model PHC-2) comprising successive steps of 93°C for 1 minute, 55°C for 1.5 minutes and 72°C for 3 minutes. An additional 0.05 units of polymerase was added at the 20 cycle stage. PCR products comprising a segment of the CFTR gene were finally subjected to gel electrophoresis (see "Molecular Cloning") in a 1.6% low-melting temperature agarose gel and PCR bands were dissected from the gel and purified on Elutip-d columns (Schleicher and Schuell, Dussel, Germany as per manufacturer's instructions). Final PCR products were ethanol precipitated and dissolved in 10mM Tris.HCl (pH7.5), 1mM EDTA (TE buffer) at a concentration of 100ng/ml.

### III. Hybridisation Analysis of Normal and Cystic Fibrosis DNA

Hybridisation reactions comprised mixtures of DNA derived from normal or cystic fibrosis afflicted individuals either with the oligonucleotide combination CFTR and oligo 1 or 2 or the combination delta 508 and oligo 1 and 2. In addition, controls comprised mixtures without human DNA. For hybridisation, 20 pmoles of oligonucleotide CFTR or delta 508 was mixed with 20 pmoles of oligo 1 or 2, 2µl (200ng) of PCR amplified human DNA (or PCR buffer blank) in 50µl of 5% formamide, 0.6M sodium chloride and 0.06M sodium citrate at pH7. Mixtures were then incubated at 37°C for 30 minutes. In one alternative reaction, a further 50µl of 0.1M TrisHCl pH8.3 containing 0.2mM dNTP mixture, 12mM MgC1₂, 80mM KCl, 20mM DTT (dithiothreitol, Sigma Chemicals, Poole, UK) and 10µCi alpha-32PdCTP (3000 Ci/mmol, Amersham International, Amersham, UK) was added followed by 40 units of AMV reverse transcriptase (Pharmacia) and the mixture was incubated a further 30 minutes at 42°C. In another alternative, 2mM dNTP was substituted for 0.2mM dNTP and 32PdCTP was omitted. In a final alternative reaction, a further 250µl of 10mM Tris.HCl pH7.5 containing 12µM dNTP mixture, 60mM MgCl₂, 100µM DTT and either 1µCi alpha-32PdCTP (3000 Ci/mmol, Amersham) or 10µCi alpha-35SdATP (6000 Ci/mmol, Amersham) was added followed by either 20 units of Klenow DNA polymerase I (United States Biochemicals) or 12 units Klenow DNA polymerase (Life Technologies, Paisley, UK) and the mixture was incubated a further 30 minutes at 30°C.

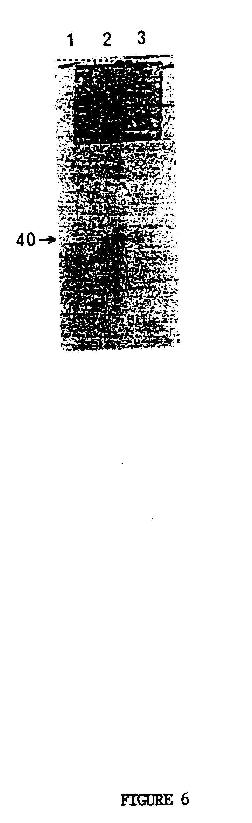

5µl aliquots of reactions were then subjected to polyacrylamide sequencing gel analysis (see "Molecular Cloning"). Figure 6 shows a typical result of such an analysis from the final alternative reaction as above including alpha-35SdATP and with oligonucleotide combination CFTR and oligo 1. Figure 6 is an autoradiograph demonstrating the extension of an unmodified oligonucleotide probe specific for the unmutated CFTR gene in the presence of the unmutated CFTR gene (lane 2) but the lack of extension of the same unmodified oligonucleotide in the presence of the delta 508 mutation of the CFTR gene (lane 1) or in the absence of CFTR gene (lane 3). Target DNA was either a PCR amplified CFTR gene, a PCR amplified delta 508 mutated CFTR gene or the products of a blank PCR reaction. This shows the expected appearance of a labelled, extended derivative of oligo 1 by hybridisation to normal DNA but not DNA containing the delta 508 CFTR gene.

Labelled nucleic acids were then subject to TCA precipitation (see "Molecular Cloning") for estimation of TCA insoluble counts. Unlabelled nucleic acids were denatured by addition of 100µl 0.2M NaOH and neutralised by adding 100µl 2M ammonium acetate. 300µl 10xSSC (SSC is 0.15M NaCl, 0.015M sodium citrate) was finally added and the mixture was filtered onto pre-wetted (20xSSC) nitrocellulose membranes (BA85 0.45 micron, Schleicher and Shuell) using a "Minifold" dot-blot apparatus (Schleicher and Schuell). Membranes were then baked at 80°C for 2 hours in a vacuum oven.

For hybridisations to filters, 10pmoles oligo 3 (table 1) was 5'-end labelled and gel purified as described in "Molecular Cloning" using T4 polynucleotide kinase (Life Technologies) and 5µl gamma-32P ATP (5000Ci/mmol, Amersham). Membranes were prehybridised for 2 hours at 68°C in 5xSSC, 5xDenhardt's reagent (see "Molecular Cloning"), 1% glycine, 0.1% SDS, 250µg/ml tRNA (Brewer's yeast, Boehringer, Lewes, UK) and 50mM sodium phosphate pH7. Hybridisation was by addition of 1 million Cerenkov cpm of probe and incubation at 37°C for 4 hours. Washes were twice for 5 minutes in 5xSSC at room temperature and once for 3 minutes at 40°C. Filters were then autoradiographed directly at -70°C using Kodak® XR-5 film.

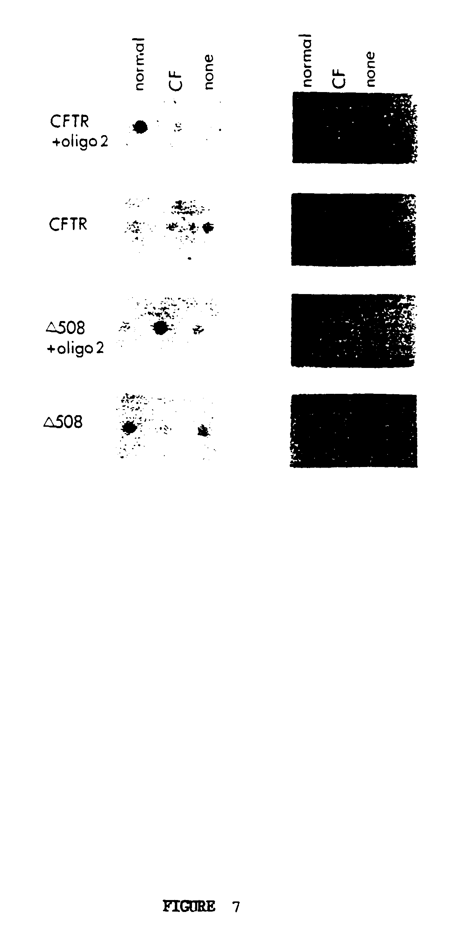

Table 2 shows the result of TCA precipitation analysis of the incorporation of label by reverse transcriptase or Klenow polymerase into homologously hybridised CFTR probe hybridised to normal DNA or delta 508 probe hybridised to DNA derived from an individual homozygous for delta 508 with little incorporation into mismatched probes or into probes in the absence of human DNA. The results are shown in Figure 7. Figure 7 is a set of autoradiographs from 2 different experiments showing the hybridisation of a phosphorous-32 labelled oligonucleotide probe specific for primer extended molecule 4 of figure 2 to nucleic acid produced following hybridisation of probes specific for the CFTR (cystic fibrosis transregulator) gene or the delta 508 derivative of this gene, in each case hybridised in conjunction with the CFTR specific probe, oligo 2 (table 1), which can act as a template for primer extension. Target DNA was either a PCR amplified CFTR gene ("normal"), a PCR amplified delta 508 mutated CFTR gene ("delta 508") or the products of a blank PCR reaction ("none"). The results of 2 separate hybridisation analyses for reverse transcriptase mediated primer extended CFTR or delta 508 probe indicates that the incorporation of label from the data of table 1 is due to specific hybridisation and extension of hybridised CFTR or delta 508 probes.

### Example 2 - Amplification of RNA and Hybridisation Detection

### I. Preparation of Oligonucleotides

Table 1 details the sequences of oligonucleotides 4 and 5 used for amplification. These were synthesised and purified as above.

### II. Amplification of Hybridised and Extended Probe

Oligonucleotides CFTR or delta 508, and oligo 2 were hybridised to CFTR DNA as in example 1. Following hybridisation, primer extension and amplification was effected either by reverse transcriptase and T7 RNA polymerase, or by Klenow polymerase and T7 RNA polymerase. For primer extension by reverse transcriptase, a 50µl mixture was added comprising 30pmoles oligo 4, 30pmoles oligo 5, 0.1M TrisHCl pH8.3, 2mM dNTP, 1mM NTP (mixture of adenosine triphosphate (ATP), uridine triphosphate (UTP), guanosine triphosphate (GTP) and cytidine triphosphate, all obtained from Pharmacia), 12mM MgCl₂, 80mM KCl, 20mM DTT, 100 units RNasin (Pharmacia), 40 units of AMV reverse transcriptase (Pharmacia) and 40 units T7 RNA polymerase (Life Technologies). 2 units RNase H (Life Technologies) was optionally added in some experiments (including those presented in table 3 and figure 5) with litle alteration to the results. In some experiments, 20µCi alpha-32P UTP (3000 Ci/mmol, Amersham) was also included in the mixture. Incubation was at 42°C for 3 hours after which radioactive samples were subject to TCA precipitation (see "Molecular Cloning") and estimation of TCA insoluble counts representing incorporation into ribonucleic acids. Nonradioactive samples were denatured by addition of 100µl 37% formaldehyde and incubation at 60°C for 15 minutes. 200µl 20xSSC was added and the samples were immobilised onto BA85 nitrocellulose membranes as described in example 1. The membrane was hybridised as above (example 1) with oligo 3 which was labelled with 32P, washed and autoradiographed as above.

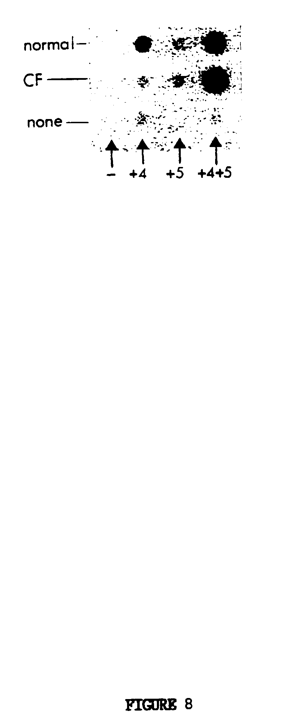

For primer extension by Klenow polymerase, a 250µl mixture was added comprising 30pmoles oligo 4, 30 pmoles oligo 5, 50mM TrisHCl pH7.75, 400µM dNTP, 500µM NTP, 5mM MgCl₂, 1mM 2-mercaptoethanol, 100 units RNasin (Pharmacia), 40 units Klenow polymerase (United States Biochemicals), 40 units T7 RNA polymerase (Life Technologies) and 20µCi alpha-32P UTP (3000 Ci/mmol, Amersham). Incubation was at 37°C for 3 hours after which radioactive samples were subject to TCA precipitation (see "Molecular Cloning") and estimation of TCA insoluble counts representing incorporation into ribonucleic acids. Table 3 shows the increased incorporation of 32P UTP into hybridisation mixtures incorporating both oligos 4 and 5 and homologously hybridising oligo CFTR or delta 508. The result also shows the reduction in 32P UTP synthesis upon omission of either oligo 4 or oligo 5. Figure 8 is an autoradiograph showing the hybridisation of a phosphorus-32 labelled oligonucleotide probe specific for primer extended molecule 4 (or RNA molecule 5) of figure 2 to nucleic acid produced following hybridisation of probe CFTR to "normal" DNA (a PCR amplified CFTR gene), delta 508 to "CF" DNA (a PCR amplified delta 508 CFTR gene) or neither CFTR nor delta 508 to "none" (a PCR blank), in each case either without oligo 2 or in conjunction with oligo 2 and either oligo 4 (+4), oligo 5 (+5) or both oligo 4 and 5 (+4+5) whereby oligos 2, 4 and 5 are defined in table 1.

This illustrates the corresponding increase in nucleic acid (primer extension by reverse transcriptase, no RNase H) homologous to labelled oligo 3 through incorporation of both oligos 4 and 5 in the amplification mixture.

### Example 3 - Amplification and Translation Mediated Detection of Hybridised Oligonucleotide Probe

### I. Preparation of M13 Transcription/Translation Template

The template for transcription and translation of a beta-galactosidase enzyme donor fragment was produced as follows: 50 pmole aliquots of oligonucleotides 5 and 6 (table 1) were heated to 90°C for 5 minutes and cooled slowly to room temperature. 2µg M13mp18 DNA (RF form, Life Technologies) was digested with EcoR1 (Life Technologies) according to manufacturer's instructions and the linearised DNA was purified by gel electrophoresis on a 1.8% low-melting temperature agarose gel and Elutip-d column chromatography. DNA was finally ethanol precipitated and dissolved in TE buffer. 0.1µg (1µl) EcoRI digested M13 DNA was mixed with the annealed oligonucleotide preparation and made up to 15µl by addition of water. 4µl of 5x ligation buffer and 1µl T4 DNA ligase (supplied together by Life Technologies) were added and the mixture incubated at 12°C for 1 hour. 10ng of the mixture was used to transform E. coli JM101 (see "Molecular Cloning") and the cloned oligonucleotide 5/6 fragment in recombinant M13 clones was confirmed by dideoxynucleotide DNA sequencing using a sequencing kit (Amersham International). RF form DNA from recombinant M13 was prepared, digested with EcoRI and gel purified as described above for M13mp18 DNA. This EcoRI digested M13mp18 DNA. This EcoRI digested M13mp18(5/6) DNA was dissolved at 100µg/ml in TE buffer.

The beta-galactosidase gene fragment was produced as follows: 50 pmoles of oligonucleotides 8 and 9 (table 1) were individually diluted with 29µl water and 10µl of 5x kinase buffer (5x = 0.25M TrisHCl pH7.6, 50mM MgCl₂, 25mM DTT, 0.5mM spermidine HCl, 0.5mM EDTA and 1mM ATP) was added. To this mixture was further added 10µCi (1µl) 5'-gamma 32P adenosine triphosphate (Amersham International, 5000Ci/mmol) and 5µl T4 polynucleotide kinase (Life Technologies). The mixture was incubated for 30 minutes at 37°C and the labelled oligonucleotides were purified on a 20% denaturing polyacrylamide gel. The purified and labelled oligonucleotides 8 and 9 were next combined and mixed with oligonucleotides 7 and 10 (table 1) in a final volume of 20µl. The mixture was heated to 90°C and slowly cooled to room temperature. 5µl of 5x ligation buffer and 1µl T4 DNA ligase (Life Technologies) were added and the mixture was incubated at 16°C overnight. The 130 base pair beta-galactosidase gene fragment was then purified on a 16% native polyacrylamide gel and dissolved in 12µl water. To this was added 0.1µg (1µl) EcoRI-digested M13mp18 (5/6) DNA, 5µl 5x ligation buffer and 2µl T4 DNA ligase (Life Technologies). The mixture was incubated at 12°C overnight and used to transform E. coli JM101 cells as above. The integrity of the beta-galactosidase fragment in "M13/bgal" recombinants was checked by sequencing as above and single-stranded DNA was prepared for the amplification reaction.

### II. Amplification of Hybridised Probes and Translation

Amplification from hybridised CFTR or delta 508 probes was achieved exactly as in example 2 except that 30pmoles of single-stranded M13/bgal DNA was substituted in place of oligo 5, and 100µM GTP and 1mM m7G(5')ppp(5')G (sodium salt, Pharmacia) was substituted for 1mM GTP. Following amplification, the mixture was phenol/chloroform extracted and ethanol precipitated (see "Molecular Cloning"). Precipitated nucleic acids were dissolved in 40µl water and 20µl of a rabbit reticulocyte lysate preparation (Life Technologies, including all amino acids) was added and incubated for 1 hour at 37°C. For complementation of beta-galactosidase, the M15 mutant of this enzyme was prepared by the method of Langley et al., J. Biol. Chem., 250, p2587-2592 and dissolved at 250 pmoles/ml in 50mM sodium phosphate pH7.2 and 5mM beta-mercaptoethanol. To the translation mixture was added 250µl of 1M sodium phosphate pH7, 2mM MgSO₄, 2mM EDTA, 0.02% NaN3 and 0.1% Tween® 20. 250µl of the M15 preparation was then added together with 1mg of O-nitrophenol beta-D-galactopyranoside (Sigma) and the mixture was incubated for 37°C for 1 hour. Samples were then placed on ice and the optical density at 414nm recorded. The results are shown in table 4 and demonstration the generation of beta-galactosidase activity through hybridisation, amplification and translation of nucleic acids to CFTR DNA.

### Example 4 - AMPLIFICATION OF DNA

### I. Preparation of Oligonucleotides

Table 1 details the sequence of oligonucleotide 11 used for amplification. This was synthesised and purified as above.

### II. Amplification of Hybridised and Extended Probe

5 pmoles oligonucleotides CFTR or delta 508, and 5 pmoles oligo 2 were mixed with 2µl CFTR DNA in 10µl (final) of 20mM TrisHCl pH8.3, 100mM KCl, 3mM MgCl₂, 400µM dNTP mixture, 0.1% Triton® X-100 (Sigma), 10µCi Alpha-32PdCTP (3000 Ci/mmol, Amersham) and 0.1 units of Taq DNA polymerase (United States Biochemical). Samples were heated to 93°C for 2 minutes and primer extended and amplified by 25 successive thermal cycles of 55°C for 1 minute, 72°C for 1.5 minutes and 93°C for 1 minute. Samples were then subjected to TCA precipitation as described in example 1.

Table 5 shows the increased incorporation of 32P dCTP into hybridisation mixtures including the amplification oligonucleotide 11.

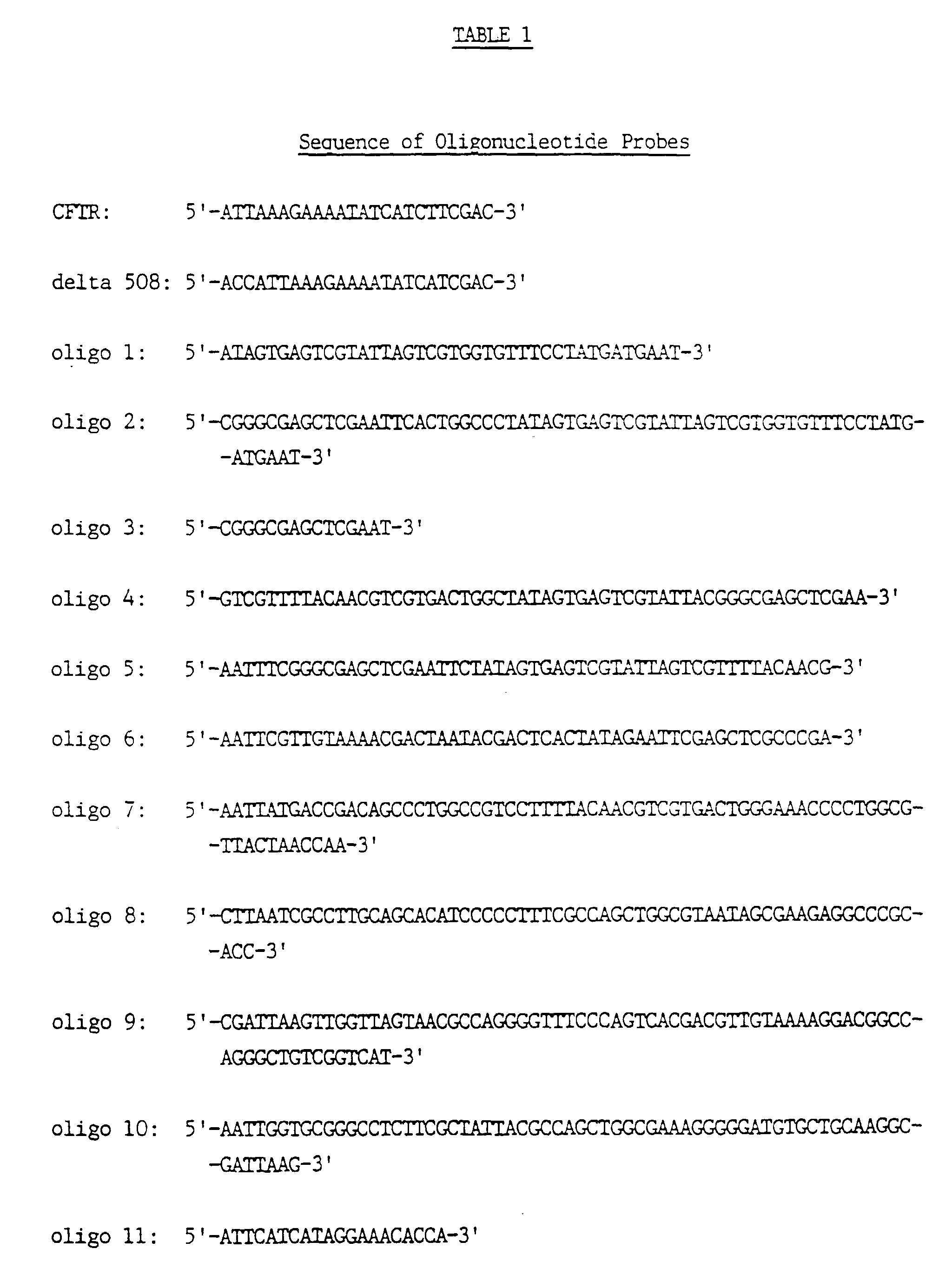

**TABLE 2**

| cpm 32P-dCTP incorporated | | | |
|---|---|---|---|
| human DNA: | normal | delta 508 | none |
| oligo probes (reverse transcriptase) | | | |
| CFTR / 2 | 18650 | 5880 | 1770 |
| delta 508 / 2 | 14510 | 19060 | 8410 |
| 2 only | 2420 | 4030 | 2080 |

| oligo probes (Klenow polymerase) | | | |
|---|---|---|---|
| CFTR / 2 | 6770 | 750 | 520 |
| delta 508 / 2 | 1370 | 2060 | 520 |
| 2 only | 190 | 50 | 100 |

**TABLE 3**

| cpm 32P-UTP incorporated | | | |
|---|---|---|---|
| human DNA: | normal | delta 508 | none |
| oligo probes (reverse transcriptase) | | | |
| CFTR/2 + oligo 4/oligo 5 | 12800 | 2050 | 1230 |
| CFTR/2 + oligo 5 | 2770 | 2420 | 520 |
| CFTR/2 + oligo 4 | 850 | 1830 | 840 |
| delta 508/2 + oligo 4/oligo 5 | 4780 | 22042 | 3420 |
| delta 508/2 + oligo 5 | 1210 | 1610 | 540 |
| delta 508/2 + oligo 4 | 510 | 90 | 290 |
| | | | |

| oligo probes (Klenow polymerase) | | | |
|---|---|---|---|
| CFTR/2 + oligo 4/oligo 5 | 6470 | 1330 | 430 |
| CFTR/2 only | 660 | 210 | 50 |

**TABLE 4**

| O.D. 414mn | | |
|---|---|---|
| human DNA: | CFTR | none |
| probes | | |
| + M13 bGal | 0.051 | 0.003 |
| - M13 bGal | 0.000 | 0.010 |

**TABLE 5**

| cpm 32P-dCTP incorporated | | | |
|---|---|---|---|
| human DNA: | normal | delta 508 | none |
| oligo probes | | | |
| CFTR/2/11 | 12540 | 3760 | 1220 |
| CFTR/2 | 950 | 930 | 45 |
| delta 508/2/11 | 5985 | 15550 | 610 |
| delta 508/2 | 180 | 200 | 20 |
| 11 only | 25 | 30 | 20 |

## Claims

1. A method of testing for the presence of a nucleic acid sequence of interest in a sample, comprising: contacting the sample with a first probe and a second probe, portions of said probes being capable of hybridising to the sequence of interest such that the probes are adjacent or substantially adjacent to one another, so as to enable other portions of the first and second probes to become annealed to each other; treating the sample so as to cause chain extension of one of the probes using part of a probe as a template; and detecting at least part of the extended probe.

2. A method according to claim 1, further comprising the use of a third probe and a fourth probe, portions of said third and fourth probes being able to hybridise respectively to at least part of the sequences of the first and second probes complementary to the sequence of interest, so as to enable other portions of the third and fourth probes to anneal to each other; and treating the sample so as to cause chain extension of the third or fourth probe using part of the third or fourth probe as a template.

3. A method according to claim 1 or 2, further comprising the ligation of pairs of probes after extension.

4. A method according to any of claims 1, 2 or 3, wherein at least part of the extended probe is amplified.

5. A method according to claim 4, wherein said at least part of the extended probe is amplified by use of a further probe.

6. A method according to claim 5, wherein the further probe comprises DNA.

7. A method according to claim 6, wherein amplification of the further probe is achieved by cycles of annealing, extension and denaturation.

8. A method according to any of the preceding claims, wherein nucleotide sequence extension is self-primed by a nucleotide sequence comprising at least some self-complementary nucleotides.

9. A method according to claim 8, wherein the extended portion of the first probe comprises at least some self-complementary nucleotides which self-prime for further extension.

10. A method according to any one of the preceding claims, wherein a self-primed nucleotide sequence is extended so as to form double stranded nucleic acid comprising a recognition site for a protein.

11. A method according to claim 10, wherein the protein is an enzyme.

12. A method according to any one of the preceding claims, further comprising: synthesising an RNA molecule comprising an RNA copy of at least part of the extended probe sequence; translating at least part of the RNA molecule so as to produce an amino acid sequence; and detecting the amino acid sequence.

13. A method according to claim 12, further comprising amplifying the RNA molecule.

14. A method according to claim 13, wherein amplification of said RNA molecule comprises RNA:DNA cycling.

15. A method according to any one of claims 12-14, comprising: translating at least part of the RNA molecule so as to produce an amino acid sequence; and detecting the amino acid sequence.

16. A method according to claim 15, wherein the amino acid sequence comprises a peptide capable of enzyme-regulating activity.

17. A method according to claim 16, wherein the amino acid sequence comprises an inactive N-terminal fragment of beta-galactosidase.

18. A method according to any one of the preceding claims, wherein a probe comprises DNA.

19. A method according to any one of the preceding claims wherein the second probe comprises at the 5' end region a sequence non-complementary to the sequence of interest and complementary to at least part of the first probe.

20. A method according to any one of the preceding claims, wherein the second probe includes a portion 30-70 nucleotides long that is non-complementary to the sequence of interest and is complementary to at least part of the first probe.

21. A method according to any one of the preceding claims, wherein the 3' end of the second probe is blocked to prevent extension.

22. A method according to any one of the preceding claims, wherein the first probe comprises at the 3' end region a sequence non-complementary to the sequence of interest and complementary to at least part of the second probe.

23. A method according to any one of the preceding claims, wherein the first probe includes a portion 2-10 nucleotides long that is non-complementary to the sequence of interest and is complementary to at least part of the second probe.

24. A method according to any one of the preceding claims wherein the first and second probes include sequences complementary to the sequence of interest which are 15-25 nucleotides long.

25. A method according to any one of the preceding claims, comprising the use of an enzyme selected from the group consisting of Thermus aquaticus (Tag) polymerase, E. coli DNA polymerase I or Klenow fragment thereof, AMV reverse transcriptase, T7 RNA polymerase and SP6 RNA polymerase.

26. A method according to any one of the preceding claims, wherein performance of the method of the invention allows nucleotide sequences differing by one base (a substitution, deletion or addition) to be distinguished.

27. Use of a kit comprising a polymerase and instructions for use in a method as defined in any one of claims 1-26.

## Patentansprüche

1. Verfahren zum Überprüfen des Vorliegens einer interessierenden Nukleinsäuresequenz in einer Probe, bei dem man die Probe mit einer ersten Sonde und einer zweiten Sonde in Kontakt bringt, wobei Teile der Sonden in der Lage sind, dergestalt an die interessierende Sequenz zu hybridisieren, daß die Sonden einander benachbart oder im wesentlichen benachbart sind, so daß andere Teile der ersten und zweiten Sonde in der Lage sind, aneinander hybridisiert zu werden; man die Probe behandelt, so daß eine Kettenverlängerung einer der Sonden verursacht wird, wobei ein Teil einer Sonde als Matrize fungiert; und man mindestens einen Teil der verlängerten Sonde detektiert.

2. Verfahren nach Anspruch 1, das ferner die Verwendung einer dritten Sonde und einer vierten Sonde, wobei Teile jener dritten und vierten Sonde in der Lage sind, jeweils an mindestens einen Teil der zur interessierenden Sequenz komplementären Sequenzen der ersten und zweiten Sonde zu hybridisieren, so daß andere Teile der dritten und vierten Sonde aneinander hybridisieren können, und das Behandeln der Probe umfaßt, so daß eine Kettenverlängerung der dritten oder vierten Sonde verursacht wird, wobei ein Teil der dritten oder vierten Sonde als Matrize fungiert.

3. Verfahren nach Anspruch 1 oder 2, das ferner die Ligation von Sondenpaaren nach Verlängerung umfaßt.

4. Verfahren nach irgendeinem der Ansprüche 1, 2 oder 3, bei dem mindestens ein Teil der verlängerten Sonde amplifiziert wird.

5. Verfahren nach Anspruch 4, bei dem der mindestens eine Teil der verlängerten Sonde unter Verwendung einer weiteren Sonde amplifiziert wird.

6. Verfahren nach Anspruch 5, bei dem die weitere Sonde DNA umfaßt.

7. Verfahren nach Anspruch 6, bei dem die Amplifikation der weiteren Sonde durch Zyklen von Hybridisierung, Verlängerung und Denaturierung erreicht wird.

8. Verfahren nach irgendeinem der vorstehenden Ansprüche, bei dem die Verlängerung der Nukleotidsequenz mittels einer mindestens einige selbstkomplementäre Nukleotide umfassenden Nukleotidsequenz durch Self-priming gestartet wird.

9. Verfahren nach Anspruch 8, bei dem der verlängerte Teil der ersten Sonde mindestens einige selbstkomplementäre Nukleotide umfaßt, die die weitere Verlängerung durch Self-priming starten.

10. Verfahren nach irgendeinem der vorstehenden Ansprüche, bei dem eine zum Self-priming fähige Nukleotidsequenz so verlängert wird, daß doppelsträngige Nukleinsäure gebildet wird, die eine Erkennungsstelle für ein Protein umfaßt.

11. Verfahren nach Anspruch 10, bei dem das Protein ein Enzym ist.

12. Verfahren nach irgendeinem der vorstehenden Ansprüche, bei dem ferner ein RNA-Molekül synthetisiert wird, das eine RNA-Kopie von mindestens einem Teil der verlängerten Sondensequenz umfaßt; mindestens ein Teil des RNA-Moleküls translatiert wird, um eine Aminosäuresequenz herzustellen; und die Aminosäuresequenz detektiert wird.

13. Verfahren nach Anspruch 12, das ferner die Amplifikation des RNA-Moleküls umfaßt.

14. Verfahren nach Anspruch 13, bei dem die Amplifikation des genannten RNA-Moleküls RNA:DNA-Zyklen umfaßt.

15. Verfahren nach irgendeinem der Ansprüche 12 bis 14, bei dem mindestens ein Teil des RNA-Moleküls translatiert wird, um eine Aminosäuresequenz herzustellen, und die Aminosäuresequenz detektiert wird.

16. Verfahren nach Anspruch 15, bei dem die Aminosäuresequenz ein Peptid umfaßt, das über Enzym-regulierende Aktivität verfügt.

17. Verfahren nach Anspruch 16, bei dem die Aminosäuresequenz ein inaktives N-terminales Fragment von β-Galactosidase umfaßt.

18. Verfahren nach irgendeinem der vorstehenden Ansprüche, bei dem eine Sonde DNA umfaßt.

19. Verfahren nach irgendeinem der vorstehenden Ansprüche, bei dem die zweite Sonde an der 5'-Endregion eine zu der interessierenden Sequenz nicht komplementäre und zu mindestens einem Teil der ersten Sonde komplementäre Sequenz umfaßt.

20. Verfahren nach irgendeinem der vorstehenden Ansprüche, bei dem die zweite Sonde einen 30-70 Nukleotide langen Teil einschließt, der zu der interessierenden Sequenz nicht komplementär ist und zu mindestens einem Teil der ersten Sonde komplementär ist.

21. Verfahren nach irgendeinem der vorstehenden Ansprüche, bei dem das 3'-Ende der zweiten Sonde blockiert ist, um eine Verlängerung zu verhindern.

22. Verfahren nach irgendeinem der vorstehenden Ansprüche, bei dem die erste Sonde an der 3'-Endregion eine zu der interessierenden Sequenz nicht komplementäre und zu mindestens einem Teil der zweiten Sonde komplementäre Sequenz umfaßt.

23. Verfahren nach irgendeinem der vorstehenden Ansprüche, bei dem die erste Sonde einen 2-10 Nukleotide langen Teil einschließt, der zu der interessierenden Sequenz nicht komplementär ist und zu mindestens einem Teil der zweiten Sonde komplementär ist.

24. Verfahren nach irgendeinem der vorstehenden Ansprüche, bei dem die erste und zweite Sonde zu der interessierenden Sequenz komplementäre Sequenzen umfassen, die 15-25 Nukleotide lang sind.

25. Verfahren nach irgendeinem der vorstehenden Ansprüche, das die Verwendung eines aus der aus Thermus aquaticus (Tag)- Polymerase, E.coli-DNA-Polymerase I oder deren Klenow-Fragment, der AMV-reversen Transkriptase, T7-RNA-Polymerase und SP6-RNA-Polymerase bestehenden Gruppe ausgewählten Enzyms umfaßt.

26. Verfahren nach irgendeinem der vorstehenden Ansprüche, bei dem es die Durchführung des erfindungsgemäßen Verfahrens erlaubt, sich nur durch eine Base unterscheidende Nukleotidsequenzen (eine Substitution, Deletion oder Addition) zu unterscheiden.

27. Verwendung eines Kits, umfassend eine Polymerase und eine Bedienungsanleitung, in einem wie in irgendeinem der Ansprüche 1 bis 26 definierten Verfahren.

## Revendications

1. Méthode de mise en évidence de la présence d'une séquence d'acides nucléiques d'intérêt dans un échantillon, comprenant : la mise en contact de l'échantillon avec une première sonde et une deuxième sonde, des portions desdites sondes étant susceptibles de s'hybrider à la séquence d'intérêt de sorte que les sondes soient adjacentes sensiblement ou adjacentes l'une à l'autre, de manière à permettre à d'autres portions des première et deuxième sondes de s'hybrider entre elles; le traitement de l'échantillon de manière à provoquer une élongation de chaîne de l'une des sondes en utilisant une partie d'une sonde comme matrice; et la détection d'au moins une partie de la sonde allongée.

2. Méthode selon la revendication 1, comprenant de plus l'utilisation d'une troisièm sonde et d'une quatrième sonde, des portions desdites troisième et quatrième sondes étant susceptibles de s'hybrider respectivement à au moins une partie des séquences des première et deuxième sondes complémentaires de la séquence d'intérêt, de manière à permettre à d'autres portions des troisième et quatrième sondes de s'hybrider entre elles; et le traitement de l'échantillon de manière à provoquer une élongation de chaîne des troisième ou quatrième sondes en utilisant une partie des troisième ou quatrième sondes comme matrice.

3. Méthode selon la revendication 1 ou 2, comprenant de plus la ligature de paires de sondes après élongation.

4. Méthode selon l'une quelconque des revendications 1, 2 ou 3, caractérisée en ce qu'au moins une partie de la sonde allongée est amplifiée.

5. Méthode selon la revendication 4, caractérisée en ce que ladite au moins une partie de la sonde allongée est amplifiée à l'aide d'une sonde supplémentaire.

6. Méthode selon la revendication 5, caractérisée en ce que la sonde supplémentaire comprend de l'ADN.

7. Méthode selon la revendication 6, caractérisée en ce que l'amplification de la sonde supplémentaire est réalisée par des cycles d'hybridation, d'élongation et de dénaturation.

8. Méthode selon l'une quelconque des revendications précédentes, caractérisée en ce que l'élongation de la séquence nucléotidique est auto-amorcée par une séquence nucléotidique comprenant au moins quelques nucléotides auto-complémentaires.

9. Méthode selon la revendication 8, caractérisée en ce que la portion allongée de la première sonde comprend au moins quelques nucléotides auto-complémentaires qui s'auto-amorcent pour une élongation supplémentaire.

10. Méthode selon l'une quelconque des revendications précédentes, caractérisée en ce qu'une séquence nucléotidique auto-amorcée est allongée de manière à former des acides nucléiques à double brin comprenant un site de reconnaissance d'une protéine.

11. Méthode selon la revendication 10, caractérisée en ce que la protéine est une enzyme.

12. Méthode selon l'une quelconque des revendications précédentes, comprenant de plus: la synthèse d'une molécule d'ARN comprenant une copie d'ARN d'au moins une partie de la séquence de la sonde allongée; la traduction d'au moins une partie de la molécule d'ARN de manière à produire une séquence d'acides aminés; et la détection de la séquence d'acides aminés.

13. Méthode selon la revendication 12, comprenant de plus l'amplification de la molécule d'ARN.

14. Méthode selon la revendication 13, caractérisée en ce que l'amplification de ladite molécule d'ARN comprend des cycles ARN:ADN.

15. Méthode selon l'une quelconque des revendications 12-14, comprenant: la traduction d'au moins une partie de la molécule d'ARN de manière à produire une séquence d'acides aminés; et la détection de la séquence d'acides aminés.

16. Méthode selon la revendication 15, caractérisée en ce que la séquence d'acides aminés comprend un peptide capable d'activité régulatrice d'enzymes.

17. Méthode selon la revendication 16, caractérisée en ce que la séquence d'acides aminés comprend un fragment N-terminal inactif de la béta-galactosidase.

18. Méthode selon l'une quelconque des revendications précédentes, caractérisée en ce qu'une sonde comprend de l'ADN.

19. Méthode selon l'une quelconque des revendications précédentes, caractérisée en ce que la deuxième sonde comprend dans la zone de l'extrémité 5' une séquence non complémentaire de la séquence d'intérêt et complémentaire d'au moins une partie de la première sonde.

20. Méthode selon l'une quelconque des revendications précédentes, caractérisée en ce que la deuxième sonde inclut une portion de 30-70 nucléotides de long qui est non complémentaire de la séquence d'intérêt et complémentaire d'au moins une partie de la première sonde.

21. Méthode selon l'une quelconque des revendications précédentes, caractérisée en ce que l'extrémité 3' de la deuxième sonde est bloquée pour empêcher l'élongation.

22. Méthode selon l'une quelconque des revendications précédentes, caractérisée en ce que la première sonde comprend dans la zone de l'extrémité 3' une séquence non complémentaire de la séquence d'intérêt et complémentaire d'au moins une partie de la deuxième sonde.

23. Méthode selon l'une quelconque des revendications précédentes, caractérisée en ce que la première sonde inclut une portion de 2-10 nucléotides de long qui est non complémentaire de la séquence d'intérêt et complémentaire d'au moins une partie de la deuxième sonde.

24. Méthode selon l'une quelconque des revendications précédentes, caractérisée en ce que les première et deuxième sondes incluent des séquences complémentaires de la séquence d'intérêt qui sont de 15-25 nucléotides de long.

25. Méthode selon l'une quelconque des revendications précédentes, comprenant l'utilisation d'une enzyme choisie dans le groupe constitué de la polymérase de Thermus aquaticus (Tag polymérase), de DNA polymérase I de E.coli ou d'un fragment de Klenow de celle-ci, de la transcriptase inverse d'AMV, de RNA polymérase de T7 et de la RNA polymérase de SP6.

26. Méthode selon l'une quelconque des revendications précédentes, caractérisée en ce que la performance de la méthode de l'invention permet la distinction entre des séquences nucléotidiques différentes d'une base (une substitution, délétion ou addition).

27. Utilisation d'un kit comprenant une polymérase et un mode d'emploi dans une méthod telle que définie dans l'une quelconque des revendications 1-26.
